Europäisches Patentamt

**European Patent Office**

**Office européen des brevets**

(19)

(11) Numéro de publication: **0 142 877**
**A2**

(12)

# DEMANDE DE BREVET EUROPEEN

(21) Numéro de dépôt: **84201468.0**

(22) Date de dépôt: **12.10.84**

(51) Int. Cl.⁴: **A 61 K 9/22**

(30) Priorité: **24.10.83 LU 85058**

(43) Date de publication de la demande:
**29.05.85 Bulletin 85/22**

(84) Etats contractants désignés:
**AT BE CH DE FR GB IT LI NL SE**

(71) Demandeur: **"PHARLYSE"**
**Boulevard Royal, 2**
**Luxembourg(LU)**

(72) Inventeur: **Deboeck, Arthur Marie**
**61 A Steenweg op Edingen**
**B-1540 Herne(BE)**

(72) Inventeur: **Baudier, Philippe**
**Avenue Blücher, 10**
**B-1410 Waterloo(BE)**

(74) Mandataire: **Schmitz, Yvon et al,**
**Bureau Gevers S.A. 7, rue de Livourne Bte 1**
**B-1050 Bruxelles(BE)**

(54) **Comprimés pharmaceutiques à libération prolongée, leur préparation et leur utilisation.**

(57) Comprimés pharmaceutiques du type à libération prolongée, dont la matrice constituant le corps des comprimés
comprend un mélange d'au moins une substance inerte avec
au moins une substance hydrophile pharmacologiquement
acceptables, leur préparation et leur utilisation.

EP 0 142 877 A2

"Comprimés pharmaceutiques à libération prolongée, leur préparation et leur utilisation".

La présente invention est relative à des comprimés pharmaceutiques à administrer par voie buccale et dont le ou les principes actifs se libèrent de façon lente et régulière, à leur préparation et à leur utilisation.

L'intérêt qui se manifeste actuellement en thérapeutique vis-à-vis de ces formes galéniques dites "à libération prolongée" provient des avantages qu'elles présentent par rapport aux formes conventionnelles, lesquelles libèrent leur principe actif de façon plus rapide ou plus brutale.

Parmi ces avantages, on citera, notamment :

- leur aptitude à produire dans l'organisme des concentrations en principe actif plus uniformes dans le temps en éliminant les effets "en dents de scie" caractérisés par des pics où la concentration en médicament excède le niveau thérapeutique et engendre des effets secondaires et des vallées où cette même concentration s'abaisse en dessous de la zone efficace.

- la possibilité qu'elles offrent d'assurer une imprégnation médicamenteuse suffisante de l'organisme pendant les périodes où les prises de médicament sont irrégulières voire absentes, par exemple pendant les périodes nocturnes et pour des médicaments à courte durée d'action.

- la diminution des variations interindividuelles des taux sanguins en médicament, obtenue par la prolongation et la régularisation de la résorption.

- la réduction du nombre de prises journalières, ce qui simplifie la posologie et entraîne une meilleure observance du patient vis-à-vis de cette posologie.

- la possibilité d'utiliser des doses élevées de médicament tout en minimisant le risque d'atteindre les concentrations toxiques, particulièrement dans le cas de médicamentsdont l'indice thérapeutique est peu élevé.

La réalisation de comprimés pharmaceutiques à libération prolongée fait actuellement appel à plusieurs techniques galéniques telles que :

- matrices hydrophiles : le principe actif est libéré lentement d'un support insoluble et gélifiable au contact des fluides digestifs. Cette matrice peut être érodable ou non.

- matrices inertes : le principe actif diffuse au-travers des canalicules d'un support inerte poreux.

- matrices lipidiques : la matrice imprégnée du principe actif s'érode plus ou moins rapidement en fonction des conditions physiologiques du tractus digestif.

- comprimés enrobés : le comprimé est revêtu d'une ou plusieurs couches de substances insolubles et poreuses au travers desquelles diffuse le principe actif.

Ces techniques sont donc pratiquement basées sur le même mécanisme : le solvant pénètre dans le comprimé et y dissout une quantité de principe actif; il se crée un gradient de concentration qui tend à expulser du comprimé la solution du principe actif. La sortie de cette solution hors du comprimé, et donc la mise du principe actif à la disposition de l'organisme est réglée soit par la viscosité du milieu, soit par les dimensions des canalicules formés dans la matrice.

Lorsqu'il s'agit de matrices érodables, qu'elles soient lipidiques ou non, la libération du principe actif est réglée par la vitesse d'érosion et par les volumes à éroder.

Dans tous les cas, la surface apparente du comprimé ou la continuité de l'enrobage influencent directement la libération du principe actif.

En dépit de leurs avantages, les formes galéniques susmentionnées présentent par conséquent l'inconvénient de modifier de façon importante la cinétique de libération du principe actif lorsque l'on fait varier leur surface ou porte atteinte à leur enrobage.

Ce phénomène est particulièrement préoccupant lorsque, pour des raisons d'adaptation de la posologie, il s'avère nécessaire de fragmenter les comprimés.

Cette pratique est néanmoins indispensable dans de nombreux cas; chez l'adulte lorsqu' une posologie doit être instaurée graduellement ou adaptée avec précision et, de façon plus impérieuse encore chez l'enfant, lorsque la posologie est tributaire du poids corporel et de la grande et continue variabilité de celui-ci.

La présente invention a pour but de remédier à cet inconvénient majeur en prévoyant un comprimé à libération prolongée dont la cinétique ne varie pas selon qu'il est entier ou fragmenté, par exemple en deux ou même en quatre parties suivant les nécessités.

A cette effet, suivant l'invention, la matrice constituant le corps du comprimé comprend un mélange d'au moins une substance inerte avec au moins une substance hydrophile pharmacologiquement acceptables .

Avantageusement, la substance inerte est constituée par un polymère acrylique ou par un mélange de tels polymères et la substance hydrophile est constituée par un dérivé cellulosique ou par un mélange de tels dérivés.

Suivant une forme de réalisation particulièrement avantageuse de l'invention, le polymère acrylique est du type Eudragit et le dérivé cellulosique est choisi dans le groupe comprenant l'éthylcellulose, l'hydroxyéthylcellulose, l'hydroxypropylcellulose, l'hydroxypropylméthylcellulose, l'hydroxybutylméthylcellulose, la carboxyméthylcellulose et ses dérivés et les mélanges de deux ou plusieurs de ces dérivés.

L'invention se rapporte également à la préparation de ces comprimés pharmaceutiques du type à libération prolongée ainsi qu'à leur méthode d'utilisation, qui consiste à administrer par la voie buccale ledit comprimé contenant une quantité thérapeutique- ment efficace de principe actif, en entier ou sous
forme d' un ou plusieurs de ses fragments.

Ainsi qu'on l'a déjà mentionné précédemment, la matrice du comprimé pharmaceutique de la présente invention est constituée, à la fois, d'au moins une substance du type polymère acrylique et d'un dérivé cellulosique. Ce nouveau type de matrice est caractérisé en ce qu'il confère, à la fois, les propriétés des matrices inertes (polymères acryliques) et les propriétés des matrices hydrophiles (dérivés cellulosiques).

Bien que, suivant ces mêmes propriétés, la cinétique de libération du médicament paraisse devoir être influencée par toute augmentation de la surface en contact avec le milieu de dissolution, la nouvelle matrice ainsi obtenue a permis d'obtenir, de façon inattendue, une cinétique ne se modifiant pas de façon significative lors de la fragmentation du comprimé.

Pour faciliter la fragmentation, le comprimé peut comporter sur une de ses faces au moins une rainure servant de guide à son fractionnement.

Le comprimé suivant l'invention est constitué d'une quantité de principe actif comprise de préférence entre 1% et 88% du poids du comprimé , d'une quantité de matrice comprise de préférence entre 11,5% et 98,5% et de la quantité nécessaire d'excipient pharmaceutiquement acceptable.

Comme principe actif utilisable, on peut citer toutes les substances solides, ou rendues telles, administrables par la voie orale dont les caractéristiques pharmacologiques et/ou pharmacocinétiques sont telles qu'il y a avantage à les présenter sous une forme à libération prolongée, et telles, par exemple, que les dérivés xanthiques, les antiinflammatoires, les β-bloquants, les inhibiteurs calciques, les antiépileptiques, les benzodiazépines, les diurétiques, les analgésiques, certaines substances hormonales, les antidépresseurs, cette énumération étant non limitative.

Comme substances aptes à former la matrice, on peut citer :

- d'une part, les polymères acryliques tels que, par exemple, les Eudragit, qu'ils soient utilisés soit sous forme sèche, soit en dispersion ou émulsion ou solution dans un véhicule aqueux ou organique.

- d'autre part, les dérivés de la cellulose tels que, par exemple, les hydroxyéthycelluloses (Natrosols), les carboxyméthylcelluloses sodiques, les hydroxypropyl-celluloses (Klucels), les hydroxyprppylméthylcelluloses (Méthocels E, F, J et K, Pharmacoat, Metolose), les hydroxybutylméthylcelluloses (Méthocels HB), les éthylcelluloses (Ethocel, Aquacoat).

Le rapport entre le ou les polymères acryliques d'une part et les dérivés cellulosiques d'autre part, est de l'ordre de 0,05/1 à 20/1, de préférence de 2/1 à 10/1.

Comme excipients pharmaceutiquement acceptables, on peut citer :

- des diluants, solubles ou insolubles, comme par exemple, le saccharose, le sorbitol, le lactose, le phosphate calcique, diverses formes de cellulose.

- des modificateurs de pH : l'acide citrique,
l'acide tartrique, le bicarbonate de soude, le carbonate de lysine.

- des antioxydants : l'acide ascorbique et ses dérivés,
les tocophérols.

- des agents conservateurs : méthyl et propylparabens,
l'acide sorbique et ses sels.

- des antistatiques : l'oxyde d'aluminium colloïdal.

- des lubrifiants : stéarate de magnésium, calcium,
laurylsulfate de sodium, huile de silicone, huile de
paraffine.

On donne ci-après des exemples non limitatifs de préparation de comprimés suivant l'invention :

Exemple 1 :

| | | |
|---|---|---|
| Théophylline | : | 200 mg |
| Lactose fin | : | 46 mg |
| Eudragit E 30 D | : | 46 mg |
| Méthocel K 100 | : | 17 mg |
| Stéarate de magnésium | : | 1,5 mg |

On mélange et tamise la théophylline, le
lactose, le Méthocel, et on granule en ajoutant
l'Eudragit E 30 D. On sèche le granulé, on le calibre
sur tamis n° 1, on ajoute le stéarate de magnésium,
mélange et comprime au poids voulu. La dureté du
comprimé terminé est supérieure à 10 Kp.

Exemple 2 :

| | | |
|---|---|---|
| Vérapamil HCl | : | 160 mg |
| Lactose fin | : | 100 mg |
| Eudragit RL | : | 6 mg |
| Eudragit L 100 | : | 10 mg |
| Klucel HF | : | 5 mg |

Stéarate de magnésium : 1,5 mg

On mélange et tamise le vérapamil, les Eudragit, le Klucel, et on granule au moyen du mélange acétone-eau (96 - 4 volumes). On sèche le granulé, le calibre sur tamis n° 1, ajoute le lactose et le stéarate, mélange et comprime au poids voulu. La dureté du comprimé terminé est supérieure à 10 Kp.

Exemple 3 :

| | | |
|---|---|---|
| Théophylline 1 $H_2O$ | : | 7,3 kg |
| Lactose fin | : | 1,5 kg |
| Eudragit E 30 D | : | 1,4 kg |
| Eudragit RS | : | 0,4 kg |
| Méthocel E5 | : | 0,3 kg |
| Stéarate de magnésium | : | 0,05 kg |
| $Al_2O_3$ colloïdal | : | 0,001 kg |

On mélange et tamise théophylline, lactose et Eudragit RS. On granule avec l'Eudragit E 30 D, on sèche et calibre sur tamis n° 1. On ajoute le Méthocel E5, le stéarate de magnésium, l'oxyde d'aluminium colloïdal et on mélange. On détermine le pourcentage de théophylline (exprimé en anhydre) dans le granulé et comprime au poids voulu pour avoir 200 mg de théophylline anhydre par comprimé. La dureté des comprimés doit être supérieure à 10 Kp.

Les comprimés préparés suivant l'exemple 3 serviront à réaliser une étude pharmacocinétique approfondie chez des volontaires sains, ainsi que des études cliniques chez des patients asthmatiques.

9.

## REVENDICATIONS.

1. Comprimé pharmaceutique du type à libération prolongée, caractérisé en ce que la matrice constituant le corps du comprimé comprend un mélange d'au moins une substance inerte avec au moins une substance hydrophile pharmacologiquement acceptables.

2. Comprimé suivant la revendication 1, caractérisé en ce que la substance inerte est constituée par un polymère acrylique ou par un mélange de tels polymères.

3. Comprimé suivant la revendication 2, caractérisé en ce que le polymère acrylique est du type Eudragit.

4. Comprimé suivant l'une quelconque des revendications 1 à 3, caractérisé en ce que la substance hydrophile est constituée par un dérivé cellulosique ou par un mélange de tels dérivés.

5. Comprimé suivant la revendication 4, caractérisé en ce que le dérivé cellulosique est choisi dans le groupe comprenant l'éthylcellulose, l'hydroxyéthylcellulose, l'hydroxypropylcellulose, l'hydroxypropyléthylcellulose, l'hydroxybutylméthyl- cellulose, la carboxyméthylcellulose et ses dérivés et les mélange de deux ou plusieurs de ces dérivés.

6. Comprimé suivant l'une quelconque des revendications 1 à 5, caractérisé en ce que le rapport pondéral de la substance inerte à la substance hydrophile est de l'ordre de 0,05/1 à 20/1.

7. Comprimé suivant la revendication 6, caractérisé en ce que le rapport pondéral de la substance inerte à la substance hydrophile est de l'ordre de 2/1 à 10/1.

8. Comprimé suivant l'une quelconque des revendications 1 à 7, caractérisé en ce qu'il comporte sur une de ses faces au moins une rainure servant de guide à son fractionnement.

9. Comprimé suivant l'une quelconque des revendications 1 à 8, caractérisé en ce qu'il comprend, comme excipient, une ou plusieurs substances choisies dans le groupe comprenant le saccharose, le sorbitol, le lactose, le phosphate calcique, les différentes formes de cellulose , l'acide citrique, l'acide tartrique, le bicarbonate de soude, le carbonate de lysine, l'acide ascorbique et ses dérivés, les tocophérols, les méthyl et propylparabens, l'acide sorbique et ses sels, l'oxyde d'aluminium colloïdal, le stéarate de magnésium, le calcium, le laurylsulfate de sodium, l'huile de silicone et l'huile de paraffine.

10. Comprimé suivant l'une quelconque des revendications 1 à 9, caractérisé en ce qu'il comprend, comme principe actif, une ou plusieurs substances choisies dans le groupe comprenant les dérivés xanthiques, les antiinflammatoires, les β-bloquants, les inhibiteurs calciques, les antiépileptiques, les benzodiazépines, les diurétiques, les analgésiques, les hormones et les antidépresseurs.

11. Comprimé suivant la revendication 10, caractérisé en ce que le principe actif constitue entre 1% et 88% du poids du comprimé.

12. Comprimé suivant l'une quelconque des revendications 1 à 11, caractérisé en ce que la matrice constitue entre 11,5% et 98,5% du poids du comprimé.

13. Procédé de préparation du comprimé suivant l'une quelconque des revendications 1 à 12, caractérisé en ce que l'on mélange le principe actif, matrice et excipient, les tamise, les granule éventuellement, calibre le granulé et le comprime pour obtenir un comprimé de poids et de dureté voulus.

14. Méthode d'utilisation du comprimé suivant l'une quelconque des revendications 1 à 12, caractérisée en ce que l'on administre par la voie buccale ledit comprimé contenant une quantité théra-peutiquement efficace de principe actif, en entier ou sous forme d'un ou plusieurs de ses fragments.

REVENDICATIONS. (Autriche)

1. Procédé de préparation d'un comprimé pharmaceutique du type à libération prolongée, caractérisé en ce que l'on mélange le principe actif, le corps du comprimé comprenant un mélange d'au moins une substance inerte avec au moins une substance hydrophile pharmacologiquement acceptables et l'excipient, les tamise, les granule éventuellement, libère le granulé et le comprime pour obtenir un comprimé de poids et de dureté voulus.

2. Procédé suivant la revendication 1, caractérisé en ce que la substance inerte est constituée par un polymère acrylique ou par un mélange de tels polymères.

3. Procédé suivant la revendication 2, caractérisé en ce que le polymère acrylique est du type Eudragit.

4. Procédé suivant l'une quelconque des revendications 1 à 3, caractérisé en ce que la substance hydrophile est constituée par un dérivé cellulosique ou par un mélange de tels dérivés.

5. Procédé suivant la revendication 4, caractérisé en ce que le dérivé cellulosique est choisi dans le groupe comprenant l'éthylcellulose, l'hydroxyéthylcellulose, l'hydroxypropylcellulose, l'hydroxypropyléthylcellulose, l'hydroxybutylméthylcellulose, la carboxyméthylcellulose et ses dérivés et les mélanges de deux ou plusieurs de ces dérivés.

6. Procédé suivant l'une quelconque des revendications 1 à 5, caractérisé en ce que le rapport pondéral de la substance inerte à la substance hydrophile est de l'ordre de 0,05/1 à 20/1.

7. Procédé suivant la revendication 6, caractérisé en ce que le rapport pondéral de la substance inerte à la substance hydrophile est de l'ordre de 2/1 à 10/1.

8. Procédé suivant l'une quelconque des revendications 1 à 7, caractérisé en ce que le comprimé comporte sur une des faces au moins une rainure servant de guide à son fractionnement.

9. Procédé suivant l'une quelconque des revendications 1 à 8, caractérisé en ce que le comprimé comprend, comme excipient, une ou plusieurs substances choisies dans le groupe comprenant le saccharose, le sorbitol, le lactose, le phosphate calcique, les différentes formes de cellulose, l'acide citrique, l'acide tartrique, le bicarbonate de soude, le carbonate de lysine, l'acide ascorbique et ses dérivés , les tocophérols, les méthyl et propylparabens, l'acide sorbique et ses sels, l'oxyde d'aluminium colloïdal, le stéarate de magnésium, le calcium, le laurylsulfate de sodium, l'huile de silicone et l'huile de paraffine.

10. Procédé suivant l'une quelconque des revendications 1 à 9, caractérisé en ce que le comprimé comprend, comme principe actif, une ou plusieurs substances choisies dans le groupe comprenant les dérivés xanthiques, les antiinflammatoires, les β-bloquants, les inhibiteurs calciques, les antiépileptiques, les benzodiazépines, les diurétiques, les analgésiques, les hormones et les antidépresseurs.

11. Procédé suivant la revendication 10, caractérisé en ce que le principe actif constitue entre 1% et 88% du poids du comprimé.

12. Procédé suivant l'une quelconque des revendications 1 à 11, caractérisé en ce que la matrice constitue entre 11,5% et 98,5% du poids du comprimé.